# EUROPEAN PATENT SPECIFICATION

(11) **EP 3 548 899 B1**
(45) Date of publication and mention of the grant of the patent: **18.10.2023**
(21) Application number: 17818438.8
(22) Date of filing: 30.11.2017
(51) Int. Cl.: G01N 33/86, C07K 16/40

(54) **RAPID SEMIQUANTITATIVE METHOD FOR DETERMINING ADAMTS-13 ACTIVITY IN A PATIENT PLASMA SAMPLE**
SCHNELLES SEMIQUANTITATIVES VERFAHREN ZUR BESTIMMUNG DER ADAMTS-13-AKTIVITÄT IN EINER PATIENTENPLASMAPROBE
PROCÉDÉ SEMI-QUANTITATIVE R RAPIDE POUR DÉTERMINER L'ACTIVITÉ D'ADAMTS-13 DANS UN ÉCHANTILLON DE PLASMA DE PATIENT

(30) Priority: 30.11.2016 EP 16201516
(43) Date of publication of application: 09.10.2019
(73) Proprietor: BERBI Biomedizinische Forschung, Entwicklung und Beratung Gesellschaft m.b.H., 1010 Vienna (AT)
(72) Inventor: VETR, Helga, 1230 Wien (AT); BINDER, Nikolaus, 1230 Wien (AT)
(74) Representative: Gassner, Birgitta
(86) International application number: PCT/EP2017/080955
(87) International publication number: WO 2018/100053

(56) References cited:
- US-A1- 2005 186 646
- US-A1- 2007 065 895
- US-A1- 2009 142 776
- BINDER NIKOLAUS B ET AL: "1399: A Rapid and Simple Assay for the Determination of Adamts-13 Activity", BLOOD; 58TH ANNUAL MEETING AND EXPOSITION OF THE AMERICAN-SOCIETY-OF-HEMATOLOGY, AMERICAN SOCIETY OF HEMATOLOGY, US; SAN DIEGO, CA, USA , vol. 128, no. 22 6 December 2016 (2016-12-06), page 1399, XP009502775, ISSN: 0006-4971 Retrieved from the Internet: URL:http://www.bloodjournal.org/content/12 8/22/1399
- ZHOU WENHUA ET AL: "An enzyme immunoassay of ADAMTS13 distinguishes patients with thrombotic thrombocytopenic purpura from normal individuals and carriers of ADAMTS13 mutations", THROMBOSIS AND HAEMOST, SCHATTAUER GMBH, DE, vol. 91, no. 4, 3 March 2004 (2004-03-03), pages 806-811, XP009107153, ISSN: 0340-6245, DOI: 10.1160/TH03-11-0675
- J.-J WU ET AL: "A rapid enzyme-linked assay for ADAMTS-13 : Rapid ADAMTS-13 assay", JOURNAL OF THROMBOSIS AND HAEMOSTASIS, vol. 4, no. 1, 1 January 2006 (2006-01-01) , pages 129-136, XP055440954, GB ISSN: 1538-7933, DOI: 10.1111/j.1538-7836.2005.01677.x
- KATO SEIJI ET AL: "Novel monoclonal antibody-based enzyme immunoassay for determining plasma levels of ADAMTS13 activity", TRANSFUSION, AMERICAN ASSOCIATION OF BLOOD BANKS, BETHESDA, MD, US, vol. 46, no. 8, 1 August 2006 (2006-08-01) , pages 1444-1452, XP002499925, ISSN: 0041-1132, DOI: 10.1111/J.1537-2995.2006.00914.X

## Description

### Field of the Invention

The present invention relates to a semiquantitative method for determining the ADAMTS-13 activity in a patient plasma sample.

### Background Art

ADAMTS-13 is a zinc metalloprotease and specifically cleaves von Willebrand factor (vWF) subunit at Tyr842-Met843 bond. If the activity of ADAMTS-13 is lowered for some reason, unusually large vWF multimers may accumulate within the blood causing thrombosis due to platelet aggregation, which in turn may lead to Thrombotic thrombocytopenic purpura (TTP).

TTP is a syndrome characterized by, for example, thrombocytopenia, hemolytic anemia and perturbing neurological dysfunction. It was formerly a poor prognostic disease with approximately 80% of patients dying within 3 months. However, currently, the prognosis has considerably improved by plasma exchange.

The possibility of using plasma ADAMTS-13 values to manage TTP patients stems from the current availability of ADAMTS-13 assays to measure ADAMTS-13 activity, ADAMTS-13 antigen and neutralizing or non-neutralizing anti-ADAMTS-13 autoantibodies.

Several assays of ADAMTS-13 activity have been developed. They are based on the cleavage of plasma-derived vWF multimers by test plasma and the direct or indirect detection of cleaved vWF by ADAMTS-13.

Direct assays involve the detection of cleavage of products either of a full-length vWF molecule or a vWF fragment that encompasses the ADAMTS-13 cleavage site:
- SDS Agarose Gel Electrophoresis and Western Blotting. In this assay purified vWF is incubated with plasma for 24 hours. Cleavage of the vWF by ADAMTS-13 takes place leading to a reduction in multimer sizes. This reduction is visualized by agarose gel electrophoresis followed by Western blotting with a peroxidase-conjugated anti-vWF antibody. The concentration of ADAMTS-13 activity in the test sample can be established by reference to a series of diluted normal plasma samples.
- SDS-PAGE and Western Blotting. This assay is similar to the above assay but involves the visualization of dimeric vWF fragments following SDS PAGE and Western Blotting. The assay is technically easier than SDS agarose gel electrophoresis and appears a very sensitive method for measuring ADAMTS-13 activity levels.
- Fluorescence Resonance Energy Transfer [FRETS] Assays. FRETS assays require two interacting partners of which one is labelled with a donor fluorophore and the other is labelled with an acceptor fluorophore. FRETS assays for ADAMTS-13 involve a chemically modified fragment of the A2 domain of vWF which spans the ADAMTS-13 cleavage site. This is readily cleaved by normal plasma but not by ADAMTS-13 deficient plasma.

Indirect assays involve the detection of cleavage of products either of a full-length vWF molecule or a vWF fragment that encompasses the ADAMTS-13 cleavage site in the A2 domain of vWF:
- Collagen Binding Assays. Normal plasma or purified vWF is incubated with the test plasma sample in the presence of BaCl₂ and 1.5M urea which denatures the vWF. VWF is cleaved by ADAMTS-13 and residual vWF is measured by its binding to collagen Type III. The bound vWF is quantitated using an ELISA assay with a conjugated anti-vWF antibody.
- Ristocetin-Induced Aggregation. This is similar to the collagen-binding assay above but residual vWF is measured by ristocetin-induced platelet aggregation using a platelet aggregometer.
- Functional ELISA Assays. In this assay, a recombinant vWF fragment is immobilized onto an ELISA plate using an antibody to a tag on the vWF. The vWF fragment encodes the A2 domain and the ADAMTS-13 cleavage site at Tyr1605-Met1606 and is tagged with S-transferase [GST]-histidine [GST-vWF73-His]. Plasma is added to the immobilized GST-vWF73-His fragment and cleavage of the immobilized fragment occurs at the ADAMTS-13 cleavage site. The cleaved vWF fragment is measured by using a second monoclonal antibody that recognizes only the cleaved vWF fragment and not the intact fragment. ADAMTS-13 activity is therefore proportional to the amount of antibody bound to the cleaved substrate.

A suitable monoclonal antibody (anti-N-10 antibody) for such a functional ELISA assay is disclosed in WO 2006/085411. However, the usual duration time of the described assay is at least 15 hours. US2005/186646 describes a rapid assay to detect ADAMTS13 activity. The usual duration time of the described rapid assay is at least 5 hours. Zhou Wenhua et al. (Thrombosis and Haemost 2004, 91(4):806-811) describe an ADAMTS13 activity assay with various step and a duration of at least 6 hours. The read-out is the optic density of 5-aminosalicyclic acid at 450 nm.

The standard laboratory test for ADAMTS-13 activity is an ELISA assay, which requires 4 hours to process. This assay tends to be performed by specialized laboratories, so samples are usually batch tested. Therefore, time needed for a physician to receive a patient's ADAMTS-13 activity result can range from 24 hours to as much as three weeks. This delay in reporting could have a severe negative impact on patient care.

Thus there remains an unmet need to provide a quick and reliable ADAMTS-13 assay in order to give patients best care.

### Summary of invention

Thus, it is an object of the present invention to provide a rapid and reliable ADAMTS-13 assay. The object is solved by the subject of the present invention.

According to one embodiment of the invention, plasma samples are pre-incubated with a substrate which is cleaved by ADAMTS-13 present in the plasma. This pre-treated sample is applied to a single test unit which contains a membrane with immobilized antibodies specific for the cleaved substrate. As the sample flows through the membrane into an absorbent pad the cleaved substrate binds to a capture antibody. A secondary biotinylated antibody is used to detect the membrane bound antibody-substrate complex. Using a Streptavidin-gold conjugate in a specific embodiment, a red color is produced. The color intensity is proportional to the level of ADAMTS-13 activity in the plasma sample and compared with a color standard, allowing semi-quantitative analysis of ADMATS-13 activity in the patient sample.

The assay can be performed at room temperature without specialized laboratory equipment and minimal training is needed to perform the test. The assay processing time is less than 30 minutes. The test is specifically designed for evaluation of a single patient sample, with a positive control run in parallel.

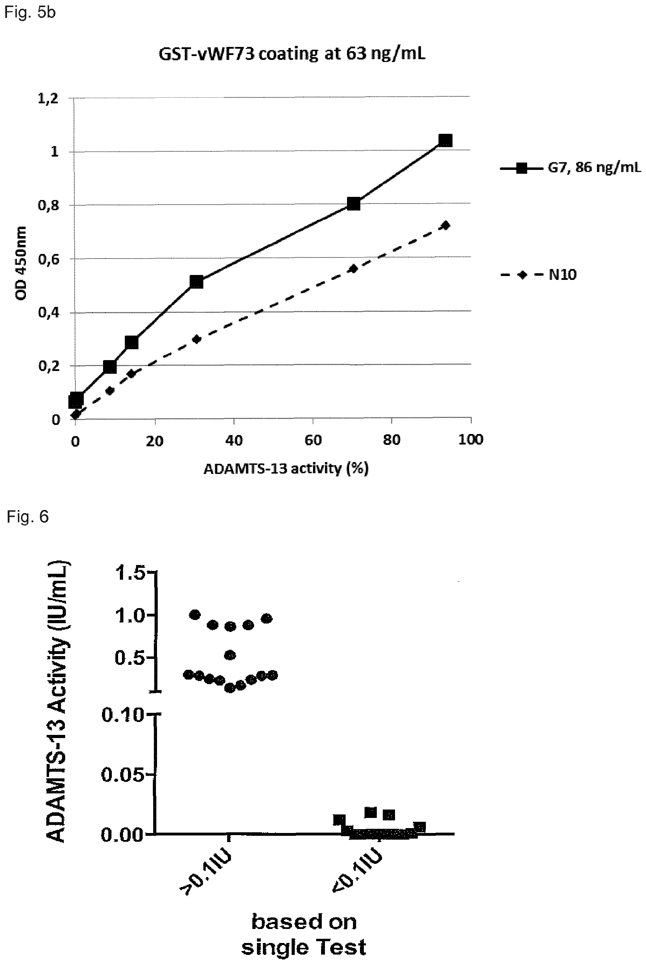

The designed assay cut-off value of 10% ADAMTS-13 activity is clinically relevant for differentiation of TTP patients. A 100% correlation was observed when TTP patient samples (n=20) were run in the TECHNOZYM ADAMTS-13 ELISA and the inventive assay, as depicted in the Fig. 6.

Described herein is a monoclonal antibody which has specific reactivity to an antigenic determinant site produced by reacting ADAMTS-13 with vWF. A further described herein is a monoclonal antibody (G7 antibody) which has specific reactivity to a vWF peptide that is cleavable by ADAMTS-13 and further by providing a method for measuring ADAMTS-13 activity using the monoclonal antibody as described herein.

Thus, the present invention relates to a method for determining theADAMTS-13 activity in a patient plasma sample, comprising the sequential steps of:
- pre-incubating said patient sample with an ADAMTS-13-cleavable peptide,
- applying the pre-treated sample of step a) to a single test unit which contains a membrane with immobilized capture antibodies specific for the ADAMTS-13-cleaved peptide, wherein the sample flows through the membrane into an absorbent pad,
- binding of said ADAMTS-13- cleaved peptide to the capture antibodies thereby forming an immobilized complex,
- adding a solution comprising a secondary biotin labelled antibody thereby forming a three-membered complex with the immobilized complex of step c),
- adding a solution comprising a chromogenic substance thereby causing a visible color change indicative of the presence of ADAMTS-13, wherein said chromogenic substance is a streptavidin-gold, and
- determining the ADAMTS-13 activity in the sample by comparing the signal intensity to a color standard card.

The invention relates to the use of a flow through technology based assay.

The invention relates to the method as described above, wherein determination of the level of ADAMTS-13 activity comprises the use of a secondary antibody labeled with a chromogenic substance which emits a detectable signal.

A further embodiment of the invention relates to the method as described above, wherein the signal intensity is proportional to the level of ADAMTS-13 activity in the patient sample.

The invention relates to the method as described above, wherein the signal is a specific color, specifically the color intensity is compared with a color standard, specifically a color standard card, allowing semi-quantitative analysis of the patient sample.

The invention relates to the method as described above, wherein the patient sample is a plasma sample.

A further embodiment of the invention relates to the method as described above, wherein the immobilized antibody is IgM antibody G7.

Described herein is an antibody or antigen binding fragment thereof which recognizes a polypeptide of SEQ ID NO:1 and which exhibits a higher affinity to ADAMTS-13 cleaved vWF when compared with the anti-N10 antibody.

As described herein the antibody exhibits a binding affinity to the polypeptide of SEQ ID NO:1 which is at least 1.5 fold higher compared to the binding affinity of the N10 antibody.

### Brief description of drawings

Fig. 1 depicts the ADAMTS-13 Rapid test principle.
Fig. 2 the test results for a non TTP Patient compared with a TTP patient.
Fig. 3 shows the results of an experiment in which the binding of G7 antibody to vWF coated plates was tested. Microtiter plates were coated with three different preparations of purified vWF (1.7 µg/mL): non-cleaved, ADAMTS-13-cleaved and elastase-cleaved vWF. In parallel, BSA was coated as control for nonspecific binding. Bound G7 was detected with anti-mouse Ig-HRP conjugate.
Fig. 4 shows the binding of G7 versus N10 antibody to ADAMTS-13 cleaved vWF. Detection of binding was performed in the same way as in Fig.3.
Fig. 5 shows the function of HRP-conjugated G7 antibody in the ADAMTS-13 ELISA as compared to the N10-HRP conjugate. The G7 antibody was purified from ascites. In Fig. 5a the GST-vWF73 substrate was used at a concentration of 250 ng/mL. In Fig. 5b GST-vWF73 was used at a concentration of 63 ng/mL.
Fig. 6 shows a graphically representation of the method comparison between single test and ELISA results.

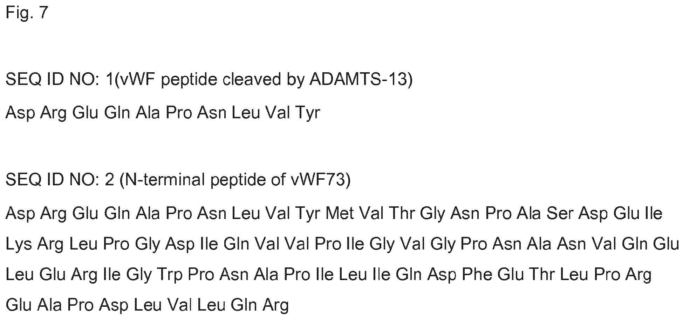

Fig. 7 depicts the sequence of the N-terminal peptide of vWF73 (SEQ ID NO:1) and of the vWF peptide cleaved by ADAMTS-13 (SEQ ID NO:2).

### Description of embodiments

The following definitions are included to provide a clear and consistent understanding of the specification and claims. As used herein, the recited terms have the following meanings. All other terms and phrases used in this specification have their ordinary meanings as one of skill in the art would understand.

The term "about" can refer to a variation of ± 5%, ± 10%, ± 20%, or ± 25% of the value specified. For example, "about 50" percent can in some embodiments carry a variation from 45 to 55 percent. For integer ranges, the term "about" can include one or two integers greater than and/or less than a recited integer at each end of the range. Unless indicated otherwise herein, the term "about" is intended to include values, e.g., weight percentages, proximate to the recited range that are equivalent in terms of the functionality of the individual ingredient, the composition, or the embodiment. The term about can also modify the end-points of a recited range.

The singular terms "a," "an," and "the" include plural reference unless the context clearly indicates otherwise. Similarly, the word "or" is intended to include "and" unless the context clearly indicates otherwise.

The term "subject" or "patient" as used herein shall refer to a warm-blooded mammalian, particularly a human being or a non-human animal.

Antibodies as described herein include polyclonal, monoclonal, humanized, bispecific, and heteroconjugate antibodies. The term "antibody" as used herein refers to polypeptides or proteins that consist of or comprise antibody domains, which are understood as constant and/or variable domains of the heavy and/or light chains of immunoglobulins, with or without a linker sequence. Polypeptides are understood as antibody domains, if comprising a beta-barrel structure consisting of at least two beta-strands of an antibody domain structure connected by a loop sequence. Antibody domains may be of native structure or modified by mutagenesis or derivatization, e.g. to modify the antigen binding properties or any other property, such as stability or functional properties, such as binding to the Fc receptors FcRn and/or Fcgamma receptor. The term "antibody" applies to antibodies of animal origin, including human species, such as mammalian, including human, murine, rabbit, rat, goat, lama, cow and horse, or avian, such as hen, which term shall particularly include recombinant antibodies which are based on a sequence of animal origin.

Antibodies may exist as intact immunoglobulins, or as modifications in a variety of forms including, for example, an Fv fragment containing only the light and heavy chain variable regions, a Fab or (Fab)'2 fragment containing the variable regions and parts of the constant regions, a single-chain antibody, and the like. The antibody may be of animal (especially mouse, goat, rabbit or rat) or human origin or may be chimeric. As used herein the term "antibody" includes these various forms, which may be produced by the modification of whole antibodies and/or synthesized *de novo* using recombinant DNA methodologies. "Monoclonal" antibodies refer to individual antibodies or populations of individual antibodies in which the antibodies are identical in specificity and affinity except for possible naturally occurring mutations that can be present in minor amounts.

These antibody fragments retain some ability to selectively bind with its antigen or receptor.

The term "label" as used herein in connection with capture antibodies refers to a detectable compound or composition which is conjugated directly or indirectly to an antibody or antibody fragment so as to generate a "labeled" antibody/antibody fragment or a "detection antibody". The label may be detectable by itself, e.g. radioisotope labels, color or fluorescent labels, gold nanoparticles or colored latex nanoparticles or, in the case of an enzymatic label, may catalyze chemical alteration of a substrate compound or composition which is detectable.

A "detectable signal" refers to a physical or chemical signal, which can be measured by visual or instrumental methods, and includes colorimetric, fluorescent, electrical and chemiluminescent signals.

Described herein is an antibody or antigen binding fragment thereof which recognizes a polypeptide of SEQ ID NO:1. In one further example the antibody exhibits a higher affinity towards ADAMTS-13 cleaved vWF when compared with the N10 antibody. A further example relates to the IgM antibody which exhibits at last a 1.2 fold higher, a 1.5 fold higher or a 2.0 fold higher binding affinity when compared to the binding affinity of the N10 antibody.

The binding affinity of the described IgM antibody is 1.2 fold, 1.3 fold, 1.4 fold, 1.5 fold, 1.6 fold, 1.7 fold, 1.8 fold, 1.9 fold, 2.0 fold of the binding affinity of the N10 antibody.

ADAMTS-13 is a zinc metalloprotease, which specifically cleaves the bond between tyrosine 1065 (Tyr1605) and methionine 1606 (Met1606) in the vWF molecule. As a result, a new antigenic determinant site is generated. It has been shown in EP 1 852 442 that antibodies, for example the anti-N-10 monoclonal antibody (N10), which recognize this antigenic determinant site, are capable of specifically binding to the cleaved vWF.

The antibody as described herein has also a specific reactivity to that antigenic determinant site. Koichi Kokame et al. (Blood 2004;103:607-612) reported that ADAMTS-13 specifically cleaves a peptidyl bond between Y1605 and M1606 in the A2 domain of vWF. In particular, it is demonstrated that a peptide composed of 73 amino acids from 1596 Asp to 1668 Arg (hereinafter referred to as "vWF73") is efficiently hydrolyzed by ADAMTS-13. The vWF73 peptide is hydrolyzed between the 10^{th} Y and the 11^{th} M by ADAMTS-13, thereby liberating a peptide composed of 10 amino acids present toward the N-terminal side from cleavage site. The peptide composed of 10 amino acids toward this N-terminal side is hereinafter also referred to as "N-10 peptide," which is a peptide having the amino acid sequence represented by SEQ ID NO:1 given in the sequence listing.

The N-10 peptide is used as an immunogen to obtain the antibody as described herein. The antibody obtained thereby has the specific reactivity to an antigenic determinant site produced by reacting the vWF-cleaving enzyme (ADAMTS-13) with von Willebrand factor (vWF) or with a peptide having the amino acid sequence represented by SEQ ID NO: 2 given in the sequence listing or at least part thereof.

The antibody thus obtained recognizes an antigenic determinant site produced by reacting ADAMTS-13 with a vWF peptide having the amino acid sequence represented by SEQ ID NO:2 but has no significant reactivity with the complete vWF molecule.

The antigenic determinant site which is found in the peptide fragment of the N-terminal side of vWF (peptide of SEQ ID NO:1) produced through hydrolysis of the peptide represented by SEQ ID NO:2 with ADAMTS-13. It is essential for the antigenic determinant site on the N-terminal side to contain tyrosine (Tyr) at the C-terminus.

Further, "has no significant reactivity" means that the antibody has no significant affinity for the complete vWF molecule. If the antibody is contacted with a complete vWF molecule only a background signal is obtained which corresponds to a signal obtained in the absence of the antibody in an ELISA.

The antibody as described herein has at least 1.2 times higher reactivity with the peptide represented by SEQ ID NO:1 compared to the monoclonal antibody N10, or at least 1.5 times higher, or at least 2.0 times higher.

The antibody as described herein is a monoclonal antibody, and is prepared by a method as is usually employed in the art. Namely, it is produced by fusing an antibody-producing cell having an affinity specific to an antigen (for example, N-10 peptide) with a myeloma cell to form a hybridoma, which is cloned to select a clone expressing an antibody specific to the respective antigen.

As an antigen, for example, the whole N-10 peptide, or the N-10 peptide with one to several amino acids deleted, substituted or added can be used as long as it contains at least tyrosine (Tyr) at the C-terminus and has antigenicity. It is believed that the antigenic determinant site is composed of at least 4 amino acids, and preferably 9 or more.

For producing the antibodies, the antigen of interest can be synthesized or prepared by a genetic engineering approach based on the selected amino acid sequence. The obtained peptide antigen can be directly dissolved or suspended in an appropriate buffer such as phosphate buffer (PBS) to use as a sensitizing antigen. The antigen is preferably crosslinked with an appropriate carrier protein such as albumin and Keyhole Limpet Hemocyanin to obtain a stronger antigenicity. The antigen solution may usually be prepared to have a concentration of approximately between 50 and 500 g/ml of an antigenic component. The animals to immunologically sensitize with the antigen of interest include mouse, rat, horse, goat, and rabbit. Mouse, in particular, BALB/c mouse is preferred. At that time, in order to immunize a subject animal to get enhanced response to the antigen, the antigen solution of interest can be mixed with an adjuvant to administer. As the adjuvant for use in the present invention, Freund's complete adjuvant (FCA), Freund's incomplete adjuvant (FIA), Ribi (MPL), Ribi (TDM), Ribi (MPL+TDM), Bordetella pertussis vaccine, muramyldipeptide (MDP), aluminum adjuvant (ALUM) and combination thereof may be exemplified. The combination with FCA at primary immunization and the combination with FIA at additional immunization are particularly preferred.

For the immunization method, injection sites, schedule and the like can be changed as appropriate in accordance with, for example, the kind of antigen to use and the presence of adjuvant to mix. For example, mice are used as the subject immunization animal to inject 0.05 to 1 mL of an antigen solution mixed with an adjuvant (10 to 200 g of antigenic component included) intraperitoneally, subcutaneously, intramuscularly or into the (tail) vein, and additionally immunized from one to four times every about four to fourteen days after the primary immunization, and then finally immunized about one to four weeks later. The antigen solution with no adjuvant contained may be administered at an increased amount of the antigen intraperitoneally. About five to six days after the additional immunization, blood is collected to measure the antibody titer. The antibody titer can be measured by a method usually used in the art according to an antibody assay described later. On approximately the 3rd to 5th day after the last immunization, splenocytes are separated from the immunized animals to obtain antibody-producing cells.

As myeloma cells, those derived from, for example, mouse, rat and human are used. For example, as mouse myeloma cells, P3X63-Ag8, P3X63-Ag8-U1, P3NS1-Ag4, SP2/0-Ag14, P3X63-Ag8-653 and the like may be exemplified, but a same type of animal, in particular a same lineage of animal is preferably used to derive the antibody-producing cells and the myeloma cells. The myeloma cells can be cryopreserved or maintained by subculturing in a common medium supplied with the serum of horse, rabbit or fetal bovine. For fusing cells, cells in the logarithmic growth phase are preferably to use.

As the method for forming hybridomas by fusing antibody-producing cells with myeloma cells, methods using, for example, polyethylene glycol (PEG), Sendai virus and an electric fusion apparatus may be exemplified. For example, in the PEG method, splenocytes and myeloma cells may be suspended in an appropriate culture medium or buffer containing approximately from 30 to 60% PEG (having an average molecular weight of 1,000 to 6,000) to mix at a mixing ratio of 1-10:1, and preferably of 5-10:1, and then allowed to react for approximately 30 sec to 3 min at a temperature of about 25 to 37 ° C and a pH of 6 to 8. After the termination of the reaction, the cells are separated from PEG solution, suspended again in the culture medium, and seeded in cell well plates to keep culturing.

The cells subjected to fusion are cultured in a selection medium to select hybridomas. The selection medium is a medium which allows parent cell strains to die and only fused-cells to proliferate, and is usually served with the hypoxanthine-aminopterin-thymidine (HAT) medium. The hybridomas are selected firstly by substituting a part of, preferably about half of the culture medium with a fresh selection medium usually 1 to 7 days after fusion, and then substituting likewise repeatedly every several days to continue culturing. Wells are observed by microscope to confirm growing colonies.

The culture supernatants may be collected and assayed for antibody to confirm whether growing hybridomas produce the desired antibodies or not. For example, when the N-10 peptide is used as an antigen, the immobilized N-10 peptide can be supplied with the culture supernatant to react, and further supplied with a secondary antibody (such as antiglobulin, anti-IgG and anti-IgM serums) labeled with, for example, fluorescent substances, enzymes and Rl to react, thereby to measure antibody titer. Wells with an appropriate antibody produced are obtained like this way. Further, monoclones are separated by a method using, for example, limiting dilution, soft agar assay and fluorescence excitation cell sorter. For example, in limiting dilution, hybridoma colonies can be serially diluted with a medium to get around 1 cell/well, and cultured to isolate a clone producing the desired monoclonal antibody. The obtained antibody-producing hybridomas can be frozen with a cryoprotective agent such as approximately 10% (v/v) DMSO or glycerin to store at -70 to -196 ° C for about half a year to semi-permanent period. The cells are rapidly thawed in a thermostat bath at around 37 ° C before use. It is desirable to wash them well before use to prevent the cryoprotective agent from remaining to exhibit cytotoxicity.

The monoclonal antibody obtained by the method described above is, for example, a monoclonal antibody which belongs to IgM class derived from mice, and is designated as "G7". The subclass of antibody produced by hybridoma can be examined by culturing the hybridoma under a general condition and analyzing the class of antibody secreted in the culture supernatant by, for example, a commercially available kit made for determining antibody class or subclass.

Of the hybridomas producing monoclonal antibodies of the present invention, hybridoma G7 lines are preferred, which produce the monoclonal antibody G7.

The method for obtaining the monoclonal antibody can be chosen appropriately from either collecting from mouse peritoneal fluid or cell culturing depending on the required amount, the nature of hybridoma or the like. Hybridomas capable of proliferating in a mouse peritoneal cavity can be obtained at a high concentration of several mg/ml from the peritoneal fluid. Hybridomas which cannot be grown *in vivo* may be obtained from a culture supernatant of cell culture. Cell culturing advantageously allows both less contamination with immunoglobulin and other contaminants which are present in the peritoneal cavity and easier purification, though it produces a smaller amount of antibody than that the *in vivo* method.

In case of obtaining monoclonal antibodies from mouse peritoneal cavity, for example, hybridomas (approximately more than 10⁶) are transplanted into the peritoneal cavities of BALB/c mice previously administered with a substance having immunosuppressive effects such as pristane (2, 6, 10, 14-tetramethylpentadecane), to collect the accumulated peritoneal fluids about 1 to 3 weeks later. For heterologous hybridomas (for example, between mouse and rat), nude mice and radiation-treated mice are preferably used.

In order to obtain antibodies from a cell culture supernatant, a culture method such as the static culture method used for maintaining cells, the high-density culture method or the spinner flask culture method are used to culture the hybridomas of interest, thereby to get the culture supernatant containing the antibody. The serum contains other antibodies and contaminants such as albumin, and so involves itself in many inconveniences for purifying the antibody. Thus, as small amount as possible of serum is desirably added into a culture medium.

It is easy to purify the monoclonal antibodies from peritoneal fluid or culture supernatant by application of a fractionation such as the salting out method using ammonium sulfate or sodium sulfate, the polyethylene glycol method, the ethanol method, the DEAE Ion Exchange Chromatography, and the gel filtration which are conventionally known as purification methods for immunoglobulin. Further, when anti-monoclonal antibody as described herein is mouse IgG antibody, purification by affinity chromatography using protein A-binding or anti-mouse immunoglobulin-binding support can be conducted and easy.

The ADAMTS rapid test may be configured as a flow through device. This device comprises a nitrocellulose membrane with the immobilized antibody for ADAMTS-13. Cleaved vWF-GST substrate, which is placed on top of an absorbent paper (2 cm² diameter). The membrane / absorbent paper are housed together in plastic casing which contains a small aperture where the reagents are added.

ADAMTS-13 activity in a sample can be rapidly measured with the antibody as described herein. vWF, which is a natural substrate for ADAMTS-13, can also be used for the measurement. Further, the peptide having the amino acid sequence represented by SEQ ID NO:2 can be used as a substrate. The substrate is preferably tagged, for example, with glutathione-S-transferase (GST) at the N-terminus of the substrate molecule by a genetic engineering approach before expression (hereinafter also referred to as "GST-vWF73 substrate"). The substrate may alternatively be labeled with an enzyme at its N-terminus, for example with horseradish peroxidase (HRP). VWF73 substrate labeled with HRP can be prepared by adding several amino acids at the N-terminus of vWF73 peptide, one of which is then mutated into cysteine (Cys) to label with HRP (Wu et al., J. Thromb. Haemost., 4, 129-136, 2006 ).

A further embodiment of the invention relates to methods for measuring ADAMTS-13 activity, wherein the monoclonal antibody which is obtained by using the N-10 peptide as immunogen (hereinafter also referred to as "G7 monoclonal antibody" or "G7 antibody") is used as the antibody according to the present invention, and the aforementioned GST-vWF73 substrate is used as the substrate.

An anti-GST antibody is immobilized on a solid support such as a microtiter plate, a tube or a magnetic particle to prepare an anti-GST antibody phase, on which GST-vWF73 substrate is fixed and immobilized. Immobilizing in this context refers to binding the antibody or antibody fragment either by chemical coupling or other non-covalent ways of attachment to a membrane with suitable physical and chemical properties to retain the binding capacity of the antibody or fragment thereof.

A reaction buffer and a sample are reacted with the immobilized substrate. Then the G7 monoclonal antibody is added and bound to the cleavage sit of the vWF73 substrate. The amount of the G7 monoclonal antibody trapped on the solid support is measured, allowing determination of ADAMTS-13 activity in the sample. The G7 monoclonal antibody is preferably labeled with a widely known labeling material.

In one embodiment of the invention an anti-GST antibody is immobilized on a solid support such as a microtiter plate, a tube or a magnetic particle to prepare an anti-GST antibody phase, on which GST-vWF73 substrate is fixed. A reaction buffer, a sample, and the G7 monoclonal antibody are simultaneously reacted with the substrate, to measure the amount of the G7 monoclonal antibody trapped on the solid support, allowing determination of ADAMTS-13 activity in the sample. The G7 monoclonal antibody is preferably labeled with a widely known labeling material.

In one further embodiment of the invention an anti-GST antibody is immobilized on a solid support such as a microtiter plate, a tube or a magnetic particle to prepare an anti-GST phase. The GST-vWF73 substrate, a reaction buffer and a sample are added and the reaction mixture is incubated. The G7 monoclonal antibody is added and the reaction mixture is incubated. The amount of the G7 monoclonal antibody trapped on the solid support is measured, thus allowing the determination of ADAMTS-13 activity in the sample. The G7 monoclonal antibody is preferably labeled with a widely known labeling material.

An anti-GST antibody is immobilized on a solid support such as a microtiter plate, a tubes or a magnetic particle to prepare an anti-GST phase. The GST-vWF73 substrate, a reaction buffer, a sample, and the G7 monoclonal antibody are simultaneously added and the reaction mixture is incubated. Thereafter, the amount of the G7 monoclonal antibody trapped on the solid support is measured thus, allowing determination of ADAMTS-13 activity in the sample. The G7 monoclonal antibody is preferably labeled with a widely known labeling material.

The method for measuring ADAMTS-13 activity using the antibody as described herein can be more specifically explained by the following methods. The IgM monoclonal antibody G7 is used according to the present invention and vWF73 is used as substrate, but the present invention is not limited to them.

The term "sample" refers to virtually any liquid sample. The sample can be derived from any desired source, such as a physiological fluid, for example, blood, saliva, ocular lens fluid, cerebral spinal fluid, sweat, urine, milk, ascites fluid, mucous, synovial fluid, peritoneal fluid, amniotic fluid or the like. The liquid test sample can be pretreated prior to use, such as preparing serum or plasma from blood, diluting viscous liquids, or the like; methods of treatment can also involve separation, filtration, distillation, concentration, inactivation of interfering components, and the addition of reagents. E.g. body fluids can be used in an *in vitro* assay. Specifically, biological samples such as blood or plasma are preferred.

Different tags can be used in place of GST and His tag to conduct the above method by using substances which are able to bind specifically to their respective tags.

ELISA is a popular format of "wet-lab" type analytic biochemistry assay that uses a solid-phase enzyme immunoassay (EIA) to detect the presence of a substance, in a liquid sample or wet sample. Thus, in one embodiment of the invention an ELISA format assay is used for determining ADAMTS-13 activity in a patient sample.

However, ADAMTS-13 activity testing using the ELISA assay format is labor-intensive and time-consuming and is only performed by specialized laboratories. As a consequence, test results are not immediately available for the physician. This delay in reporting could have a negative impact on patient care.

Novel methods under the point-of-care testing (POCT) format provide advantages in terms of test simplicity and speed. POCT, or bedside testing is defined as medical diagnostic testing at or near the point of care - that is, at the time and place of patient care. Many point-of-care test systems are realized as easy-to-use membrane-based test strips, often enclosed by a plastic test cassette.

Flow-through tests or immunoconcentration assays are a type of diagnostic assays that allows users to test for the presence of a biomarker, usually a specific antibody, in a sample such as blood. They are a type of point of care test, a test designed to be used by a healthcare provider at patient contact. Point of care tests often allow for rapid detection of a specific biomarker without specialized lab equipment and training; this aids in diagnosis and allows therapeutic action to be initiated more quickly.

The terms "flow through assay", "flow through technology based assay" and "flow through test" can be used interchangeably herein. Flow-through technology based assays are by principle binding assays. In practice they are mostly applied to detect the interaction of an antibody, as from a sample of the test subject's blood, with immobilized antigens, resulting in the formation of an antigen-antibody complex. However, other types of capture-assays are technically feasible, including small molecule capture-assays or antigen tests.

Flow-through tests typically come in the form of cassettes divided into four parts: an upper casing, a reactive membrane panel, an absorbent panel, and a lower casing. To perform a test, a diluted sample is applied to the reactive membrane panel and flows through to the absorbent pad, with the target analyte being captured in the membrane. The membrane is then washed to remove unbound, non-target molecules, washed again with a solution containing a signal reagent, and washed again to remove unbound signal reagent. If the analyte was present in the original sample, then by the end of this process it should be bound to the membrane, with the signal reagent bound to it, revealing the presence of the analyte on the membrane.

A lateral flow-based assay comprises a pre-incubation step of the patient samples with a substrate. Said mixture is then applied to a test strip that contains a membrane comprising a test zone or line with a capture antibody. Result of the test can be detected due to e.g. releasable streptavidin-coated gold particles causing the presence or absence of a visible red line formed at the test zone or line. The term "flowing through" or "flow through" used herein in connection with lateral flow based assays refers to a flow of the cleaved peptide along the membrane, wherein the peptide then binds to the capture antibody.

The membrane as used herein can be any membrane wherein antibodies can be immobilized and which have a pore size which allows the cleaved peptides to flow through. Specifically said membranes are nitrocellulose membranes.

The present inventors have found a flow immunoassay structure that overcomes all prior art drawbacks. More specifically, the flow immunoassay of the present invention is small, simple, fast, easy to use and cost-effective. In addition, it does not need a qualified operator. It dramatically reduces operating times and resources. Therefore, the present invention allows a rapid semiquantitative analysis of patient samples.

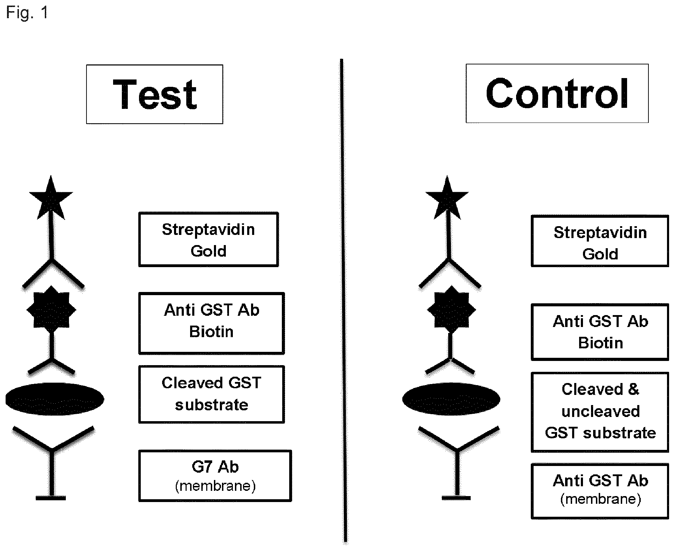

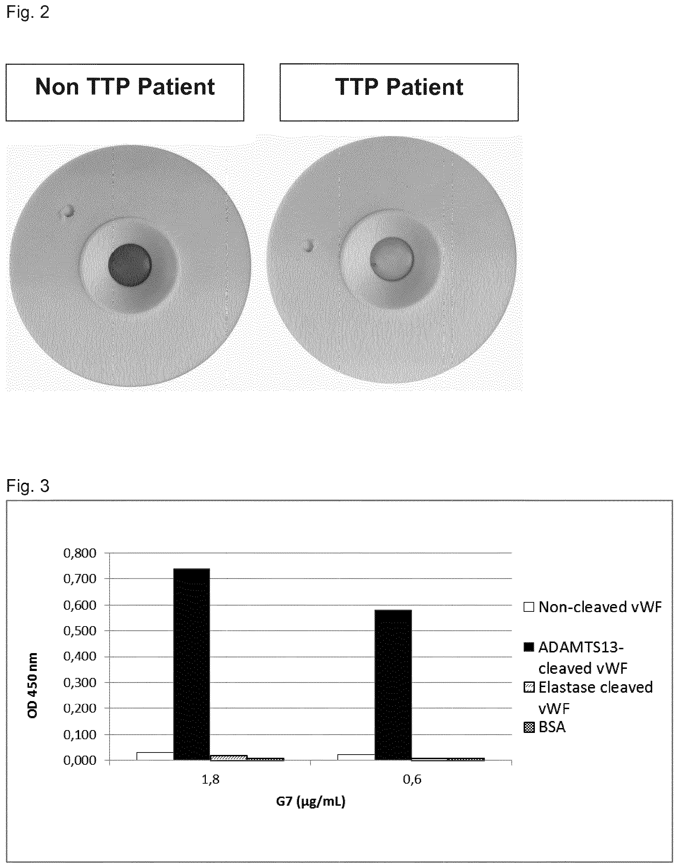

The rapid test principle is illustrated in Fig 1. Plasma samples are pre-incubated with a recombinant glutathione-S-transferase (GST)-tagged vWF substrate for 20 minutes at room temperature. The vWF substrate is cleaved by ADAMTS-13 present in the plasma. The pre-treated sample is applied to the test device via aperture, which contains a membrane with an immobilized capture antibody, e.g., the G7-antibody, specific for the cleaved substrate, while a corresponding control well contains capture antibodies specific for both uncleaved and cleaved substrate. As the sample flows through the membrane into an absorbent pad, the uncleaved substrate binds to the immobilized antibody. A secondary biotinylated anti-GST antibody is used to detect the membrane-bound complex and is revealed by reactivity with a streptavidin-gold conjugate, producing a red color. The color intensity is proportional to the level of ADAMTS-13 activity in the plasma sample and is compared with a color standard card, allowing qualitative analysis of the patient sample (< or > 0.1 IU/mL, corresponding to a cut-off value of 10% ADAMTS13 activity), as illustrated in Fig 2.

In common with other WHO IS for plasma proteins it was therefore proposed that an International Unit (IU) should be adopted for ADAMTS13 activity where 0.91 IU/mL is equivalent to the concentration of ADAMTS13 in pooled normal plasma (WHO 1st International Standard ADAMTS13 Plasma; NIBSC code: 12/252).

Thus, a further embodiment of the invention relates to a flow through assay, wherein patient samples are pre-incubated with a substrate, e.g. vWF, which is cleaved by ADAMTS-13 present in the plasma.

"Pre-incubation" refers to contacting a sample with an ADAMTS-13 cleavable peptide for a time period sufficient for ADAMTS-13 protease to degrade the cleavable protein or polypeptide. The time period depends on the concentration of protease and the amount of sample and can be determined by the skilled person without undue burden. According to an embodiment of the invention, the samples are pre-incubated for at least 1 min, 5 min, 10 min or more. More specifically the samples are pre-incubated at room temperature for about 1 min, specifically for about 2 min, specifically about 5 min

This pre-treated sample is applied to the single test unit which contains a membrane with immobilized antibodies specific for the cleaved substrate (e.g. G7 antibody). As the sample flows through the membrane into an absorbent pad the cleaved substrate binds to capture antibody. A secondary biotinylated antibody is used to detect the membrane bound complex. Using a Streptavidin-gold conjugate a red colour is produced. The colour intensity is proportional to the level of ADAMTS-13 activity in the plasma sample and compared with a colour standard card, allowing semi-quantitative analysis of the patient sample.

The ADAMTS-13 rapid test was validated with the Technozym ADAMTS-13 ELISA (standard method to determine ADAMTS-13 activity). For the rapid test the patient samples were classified as < or > 0.1 IU/mL which is the cut-off of this assay (see Fig. 6). Whereas the ELISA results were quantifiable by using a calibrator set traceable to the international standard for ADAMTS-13.

The rapid test system is specifically designed for evaluation of a single patient sample, with a positive control run in parallel. Thus, use of the rapid test system has several advantages when compared with standard ELISA test, e.g. eliminates the delays associated with batch analyses with the standard ELISA tests. The novel assay can be performed at room temperature without specialized laboratory equipment. In addition, minimal operator training is required, and the assay processing time is under 30 minutes compared to 4 hours with the standard ELISA tests. Therefore the novel ADAMTS-13 test system is an ideal screening tool for TTP diagnosis.

### Examples

The Examples which follow are set forth to aid in the understanding of the invention but are not intended to, and should not be construed to limit the scope of the invention in any way. The Examples do not include detailed descriptions of conventional methods. Such methods are well known to those of ordinary skill in the art.

### Example 1- Production of hybridoma and G7 monoclonal antibody

### Method for producing hybridoma

Generation of mouse monoclonal antibodies was done using standard procedures. Briefly, the peptide -DREQAPNLVY (derived from the vWF A domain, aa 1596-1605) was used as antigen and coupled to KLH or BSA. Mice were immunized with either one of the two conjugates. Screening of primary clones was done by ELISA, in a three step procedure. In the first step, supernatants were screened on conjugate (peptide- KLH or peptide-BSA) coated plates. Supernatant of peptide-KLH immunization was incubated on peptide-BSA coated plates and vice versa. In the second step, positive clones from step 1 were screened on plates coated with ADAMTS-13-cleaved vWF. In the third step, positive clones from step 2 were tested on non-cleaved or Elastase-cleaved vWF. Clone G7 reacted positive only on ADAMTS-13 cleaved vWF and did not bind to non-cleaved or Elastase-cleaved vWF.

### Example 2 - Determining specificity of G7 antibody

The G7 producing hybridoma clone was recloned three times, grown in culture medium, and then inoculated into mice for ascites fluid development. G7 Antibody was purified from this fluid by standard ammonium sulfate precipitation. Specificity of this G7 preparation was verified using different preparations of vWF fragments as described in Example 1. As a negative control bovine serum albumin (BSA) was used and G7 bound to those various vWF preparations was detected by a HRP-conjugated anti-mouse Ig antibody.

**Table 1 - Binding of G7 to cleaved vWF**

| | OD 450 nm | |
|---|---|---|
| coated protein | G7 [1.8 µg/ml] | G7 [0.6 µg/ml] |
| non-cleaved vWF [1.7 µg/ml] | 0.030 | 0.020 |
| non-cleaved vWF [0.85 µg/ml] | 0.020 | 0.020 |
| ADAMTS-13-cleaved vWF [1.7 µg/ml] | 0.740 | 0.580 |
| ADAMTS-13-cleaved vWF [0.85 µg/ml] | 0.400 | 0.290 |
| elastase cleaved vWF [1.7 µg/ml] | 0.020 | 0.010 |
| elastase cleaved vWF [0.85 µg/ml] | 0.020 | 0.010 |
| BSA [30 µg/ml] | 0.010 | 0.010 |
| BSA [15 µg/ml] | 0.010 | 0.010 |

As shown in Table 1 and Fig. 3, the obtained antibody G7 binds specifically and in a concentration dependent manner to ADAMTS-13 cleaved vWF. It does not bind to non-cleaved or elastase cleaved vWF or unrelated protein (BSA)

### Example 3 - Comparing binding affinities of G7 and N10

The assay protocol as described in Example 1 was applied for comparison of the N10 antibody and for the obtained antibody G7.

**Table 2 - Binding of N10 to cleaved vWF**

| | N10 [1.8 µg/ml] | N10 [0.6 µg/ml] |
|---|---|---|
| ADAMTS-13 -cleaved vWF [1.7 µg/ml] | 0.470 | 0.410 |
| ADAMTS-13 -cleaved vWF [0.85 µg/ml] | 0.240 | 0.210 |

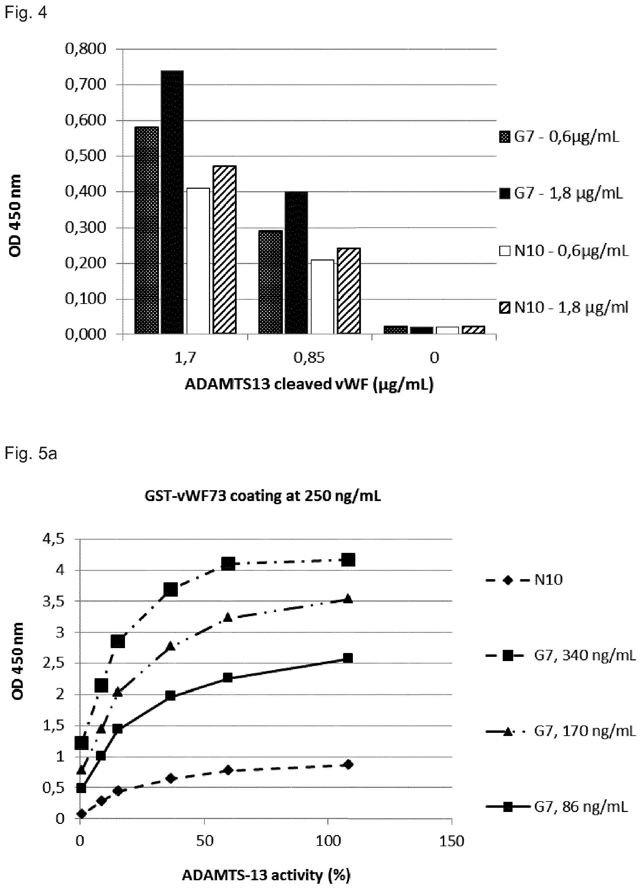

The novel monoclonal antibody G7 binds with higher affinity to ADAMTS-13 cleaved vWF than the N10 antibody when applied in the same concentration (see also Fig. 4).

### Example 4 - Comparing HRP conjugates of G7 and N10 in the ADAMTS13 activity ELISA

The method for measuring ADAMTS13 activity using the antibody as described herein can be more specifically explained by the following methods. The G7 monoclonal antibody as characterized in example 3 is used as the antibody together with a vWF fragment which is used as ADAMTS13 substrate. This substrate is a recombinant polypeptide encoding a 73 amino acid sequence of the vWF A2 domain which contains the ADAMTS13 cleavage site at Tyr1605-Met1606 and which is tagged with Glutathion S-transferase [GST]-at the N-Terminus and with a Histidine tag at the C-Terminus [GST-vWF73-[His]. This GST-vWF73-His substrate, a reaction buffer and a sample are reacted in a container such as a test tube to prepare a reaction mixture, which is then reacted with a HRP labelled G7 monoclonal antibody.

In detail, ELISA test strips were coated with a monoclonal anti GST-antibody. 100 *µ*L of GST-vWF73 substrate solutions were added to the anti-GST coated wells and incubated for 60 min at room temperature. After a washing step 100 *µ*L of calibrators, control plasmas and samples were added to the test wells, respectively and incubated for 30 min at room temperature. After a washing step the HRP labelled G7 monoclonal antibody was added and incubated for 60 min at room temperature. After a washing step a color reagent was added to the wells. After incubation for 30 min at room temperature the reaction was stopped and the ADAMTS13 activity determined by measuring the absorption at wavelength of 450 nm.

The method as described above was applied for comparison of the G7 antibody with the N10 antibody in form of their HRP conjugates.

**Table 3 - Comparison of G7 and N10 antibody in ADAMTS-13 activity ELISA**

| Calibrators (% ADAMTS-13 activity) | N10 | G7 [340 ng/ml] | G7 [170 ng/ml] | G7 [86 ng/ml] |
|---|---|---|---|---|
| 100.3 | 0.859 | 4.169 | 3.535 | 2.57 |
| 59.8 | 0.772 | 4.096 | 3.227 | 2.221 |
| 36.3 | 0.638 | 3.689 | 2.759 | 1.953 |
| 15.2 | 0.439 | 2.854 | 2.028 | 1.425 |
| 8.5 | 0.28 | 2.137 | 1.428 | 0.992 |
| 0.5 | 0.063 | 1.223 | 0.764 | 0.483 |
| blank | 0.032 | 1.19 | 0.87 | 0.512 |

HRP conjugates of the antibodies and the standard GST-vWF73-His concentration of 250 ng/mL was used.

Using the G7 conjugate in the ADAMTS-13 activity ELISA under standard conditions, i.e. conditions optimized for the N10 antibody, much higher OD values are obtained than with the N10 conjugate, as expected from results in Example 3. The resulting calibration curve resembles a saturation curve, indicating an excess of G7 conjugate even at a conjugate concentration as low as 86 ng/mL. This shows the superior binding affinity of G7 over N10 antibody (see also Fig. 5a).

### Example 5 - Comparing coating concentrations of GST-vWF73 on ADAMTS-13 activity

In order to optimize the ELISA for G7 the coating concentration of GST-vWF73 was adjusted and both, N10 and G7 were tested with this modification of the assay. Otherwise, the assay was performed according to the protocol described in Example 4.

**Table 4 - Comparison of G7 and N10 antibody in ADAMTS-13 activity ELISA**

| GST-vWF73 63 ng/mL | | |
|---|---|---|
| Calibrators (% ADAMTS13 activity) | N10 | G7 [86 ng/ml] |
| 93.9 | 0.716 | 1.036 |
| 70.6 | 0.558 | 0.802 |
| 30.7 | 0.298 | 0.514 |
| 14.2 | 0.167 | 0.288 |
| 8.7 | 0.104 | 0.196 |
| 0.35 | 0.020 | 0.077 |
| 0 | 0.015 | 0.067 |
| blank | 0.07 | 0.027 |

By reducing the coating concentration of the GST-vWF73-His substrate linear calibration curves can be obtained for both the N10 and the G7 antibody. Under these conditions the calibration curve using G7 antibody has a higher slope than that using N10 antibody, which is a pre-requisite for a more sensitive assay (see also Fig. 5b).

## Claims

1. A rapid semiquantitative method for determining ADAMTS-13 activity in a patient plasma sample, comprising the steps of:
a) pre-incubating said patient sample with an ADAMTS-13-cleavable peptide,
b) applying the pre-treated sample of step a) to a single test unit which comprises a membrane with an immobilized capture antibody specific for the ADAMTS-13-cleaved peptide, wherein the sample flows through said membrane into an absorbent pad,
c) binding of said ADAMTS-13-cleaved peptide to the capture antibody thereby forming an immobilized complex,
d) adding a solution comprising a secondary biotin labelled antibody thereby forming a three-membered complex with the immobilized complex of step c),
e) adding a solution comprising a chromogenic substance thereby causing a visible color change indicative of the presence of ADAMTS-13, wherein said chromogenic substance is a streptavidin-gold, and
determining ADAMTS-13 activity in said patient sample by comparing the signal intensity to a color standard card.

2. The method according to claim 1, wherein the color intensity is proportional to the level of ADAMTS-13 activity in the patient sample, and wherein the color intensity is compared with a color standard, allowing semi-quantitative analysis of the patient sample.

3. The method according to claim 1 or 2, wherein the immobilized antibody is IgM antibody G7, and wherein the secondary antibody is an anti-GST-antibody.

## Patentansprüche

1. Schnelles semiquantitatives Verfahren zum Bestimmen der ADAMTS-13-Aktivität in einer Patientenplasmaprobe, umfassend die Schritte:
a) Vorinkubieren der Patientenprobe mit einem durch ADAMTS-13 spaltbaren Peptid,
b) Aufbringen der vorbehandelten Probe aus Schritt a) auf eine einzige Testeinheit, die eine Membran mit einem immobilisierten Fängerantikörper umfasst, der für das durch ADAMTS-13 gespaltene Peptid spezifisch ist, wobei die Probe durch die Membran in ein Saugkissen fließt,
c) Binden des durch ADAMTS-13 gespaltenen Peptids an den Fängerantikörper, wodurch ein immobilisierter Komplex gebildet wird,
d) Zugeben einer Lösung, die einen biotinmarkierten Sekundärantikörper umfasst, wodurch ein dreigliedriger Komplex mit dem immobilisierten Komplex aus Schritt c) gebildet wird,
e) Zugeben einer Lösung, die eine chromogene Substanz umfasst, wodurch ein sichtbarer Farbwechsel hervorgerufen wird, der auf das Vorhandensein von ADAMTS-13 hinweist, wobei die chromogene Substanz ein Streptavidin-Gold ist, und
Bestimmen der ADAMTS-13-Aktivität in der Patientenprobe durch Vergleichen der Signalintensität mit einer Farbstandardkarte.

2. Verfahren nach Anspruch 1, wobei die Farbintensität proportional zum Niveau der ADAMTS-13-Aktivität in der Patientenprobe ist und wobei die Farbintensität mit einem Farbstandard verglichen wird, was eine semiquantitative Analyse der Patientenprobe erlaubt.

3. Verfahren nach Anspruch 1 oder 2, wobei der immobilisierte Antikörper der IgM-Antikörper G7 ist und wobei der Sekundärantikörper ein Anti-GST-Antikörper ist.

## Revendications

1. Méthode semi-quantitative rapide permettant de déterminer l'activité ADAMTS-13 dans un échantillon de plasma de patient, comprenant les étapes de :
a) pré-incubation dudit échantillon de patient avec un peptide clivable par ADAMTS-13,
b) application de l'échantillon pré-traité de l'étape a) sur une unité de test unique qui comprend une membrane avec un anticorps de capture immobilisé spécifique pour le peptide clivé par ADAMTS-13, dans laquelle l'échantillon s'écoule à travers ladite membrane dans un tampon absorbant,
c) liaison dudit peptide clivé par ADAMTS-13 à l'anticorps de capture formant ainsi un complexe immobilisé,
d) ajout d'une solution comprenant un anticorps secondaire marqué à la biotine formant ainsi un complexe à trois chaînons avec le complexe immobilisé de l'étape c),
e) ajout d'une solution comprenant une substance chromogène provoquant ainsi un changement de couleur visible indiquant la présence d'ADAMTS-13, dans lequel ladite substance chromogène est une substance streptavidine-or, et
détermination de l'activité ADAMTS-13 dans ledit échantillon de patient en comparant l'intensité de signal à une carte de normes de couleurs.

2. Méthode selon la revendication 1, dans laquelle l'intensité de couleur est proportionnelle au niveau d'activité ADAMTS-13 dans l'échantillon de patient, et dans laquelle l'intensité de couleur est comparée à une norme de couleur, permettant une analyse semi-quantitative de l'échantillon de patient.

3. Méthode selon la revendication 1 ou 2, dans laquelle l'anticorps immobilisé est un anticorps IgM G7, et dans laquelle l'anticorps secondaire est un anticorps anti-GST.
